(19) Europäisches Patentamt
European Patent Office
Office européen des brevets

(11) **EP 3 275 371 A1**

(12) **EUROPEAN PATENT APPLICATION**

(43) Date of publication:
**31.01.2018 Bulletin 2018/05**

(51) Int Cl.:
**A61B 5/18** *(2006.01)*          **A61B 5/024** *(2006.01)*
**A61B 5/04** *(2006.01)*          **A61B 5/0456** *(2006.01)*

(21) Application number: **17181158.1**

(22) Date of filing: **13.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.07.2016 JP 2016150661**

(71) Applicant: **Fujitsu Limited**
**Kawasaki-shi, Kanagawa 211-8588 (JP)**

(72) Inventors:
• **Nakano, Yasuhiko**
  **90449 Nuernberg (DE)**
• **Sano, Satoshi**
  **Kanagawa, 211-8588 (JP)**
• **Senokuchi, Satoshi**
  **Fukuoka, 814-8588 (JP)**
• **Kubota, Eiichiro**
  **Kanagawa, 211-8588 (JP)**

(74) Representative: **Hoffmann Eitle**
**Patent- und Rechtsanwälte PartmbB**
**Arabellastraße 30**
**81925 München (DE)**

(54) **DROWSINESS DETECTION PROGRAM MEDIUM, DROWSINESS DETECTION METHOD, AND DROWSINESS DETECTION APPARATUS**

(57)    A drowsiness detection method that causes a computer to execute a process, the process includes: performing frequency analysis on heartbeat data obtained from a subject; and determining a state of the subject by calculating entropy in a predetermined frequency bandwidth with a peak as a reference, where the peak is within a predetermined frequency range of frequency distribution obtained from the frequency analysis result, determining that the subject is in a relaxation state in a case where the calculated entropy is less than a predetermined threshold value, and determining that the subject is in a drowsiness state in a case where the entropy is equal to or greater than the threshold value.

## FIG. 1

## Description

FIELD

The embodiments discussed herein are related to a drowsiness detection program medium, a drowsiness detection method, and a drowsiness detection apparatus.

BACKGROUND

Whereas the total number of traffic accidents has been decreasing year by year, accidents caused by human errors have not decreased so much. One reason for the accidents caused by human errors is drowsiness while driving. For this reason, a technique that avoids accidents in advance by outputting a warning to a driver based on an arousal level while driving, is preferably considered.

For example, as a technique in related art that is used for determining the arousal level, there is a technique that determines the arousal level of the driver based on a change of maximum points in frequency ranges of low frequency (LF) and high frequency (HF) of power spectral density calculated by frequency analysis of a heartbeat signal of the driver. In such a technique in related art, in a case where the arousal level is low, it is determined that the driver is in a drowsiness state. Examples of related art include Japanese Laid-open Patent Publication No. 2007-289540 and Japanese Laid-open Patent Publication No. 7-108847.

However, the low arousal level state also includes a state other than the drowsiness state of the driver. For this reason, in the technique in the related art described above, in some cases, the drowsiness state of the driver may not be accurately determined.

In an aspect, an object of the embodiments disclosed herein is to provide a drowsiness detection program, a drowsiness detection method, and a drowsiness detection apparatus capable of accurately estimating a drowsiness state of a subject.

SUMMARY

According to an aspect of the invention, a drowsiness detection method that causes a computer to execute a process, the process includes: performing frequency analysis on heartbeat data obtained from a subject; and determining a state of the subject by calculating entropy in a predetermined frequency bandwidth with a peak as a reference, where the peak is within a predetermined frequency range of frequency distribution obtained from the frequency analysis result, determining that the subject is in a relaxation state in a case where the calculated entropy is less than a predetermined threshold value, and determining that the subject is in a drowsiness state in a case where the entropy is equal to or greater than the threshold value.

BRIEF DESCRIPTION OF DRAWINGS

FIG. 1 is a functional block diagram illustrating a configuration of a drowsiness detection apparatus according to the present example;
FIG. 2 is a diagram illustrating an example of heartbeat signal data;
FIG. 3 is a diagram illustrating an example of heartbeat interval change data;
FIG. 4 is a diagram illustrating a relationship between a frequency and a power spectral density;
FIG. 5 is a diagram for explaining processing of evaluating an arousal level;
FIG. 6 is a diagram for explaining features of frequency bands;
FIG. 7A is a diagram illustrating an example of frequency distribution;
FIG. 7B is a diagram illustrating an example of a frequency range in the frequency distribution that is used for calculating entropy;
FIG. 7C is a diagram illustrating another example of a frequency range in the frequency distribution that is used for calculating entropy;
FIG. 8A is a diagram illustrating an example of the frequency distribution in a case where a subject is in a normal condition state;
FIG. 8B is a diagram illustrating an example of the frequency distribution in a case where a subject is in a bad condition state;
FIG. 9 is a flowchart illustrating an example of a procedure of drowsiness detection processing; and
FIG. 10 is a diagram illustrating an example of a computer that executes a drowsiness detection program.

DESCRIPTION OF EMBODIMENTS

Hereinafter, examples of a drowsiness detection program, a drowsiness detection method, and a drowsiness detection apparatus according to the embodiments disclosed herein will be described in detail with reference to the drawings. The embodiments disclosed herein are not limited to the examples.

First Example

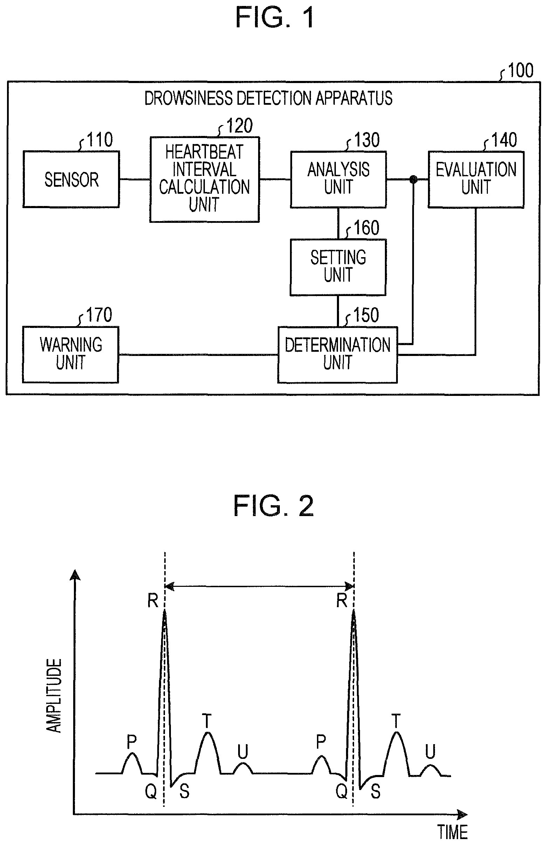

FIG. 1 is a functional block diagram illustrating a configuration of a drowsiness detection apparatus according to the present example. As illustrated in FIG. 1, the drowsiness detection apparatus 100 includes a sensor 110, a heartbeat interval calculation unit 120, an analysis unit 130, an evaluation unit 140, a determination unit 150, a setting unit 160, and a warning unit 170.

The sensor 110 is a sensor that detects a heartbeat signal of a subject. For example, the subject corresponds to a driver of a vehicle. For example, the sensor 110 is provided in a steering wheel or the like of the vehicle. In the present example, as an example, a case of detecting a heartbeat signal will be described. In some cases, the sensor 110 may detect a pulse signal of the subject. The heartbeat signal and the pulse signal are examples of biological signals. The sensor 110 outputs data of the heartbeat signal to the heartbeat interval calculation unit 120. In the following description, the data of the heartbeat signal is referred to as heartbeat signal data. In the present example, the heartbeat signal data corresponds to heartbeat data.

FIG. 2 is a diagram illustrating an example of the heartbeat signal data. As illustrated in FIG. 2, the heartbeat signal data has waveforms called as P, Q, R, S, T, and U waves. For example, in FIG. 2, the horizontal axis indicates time, and the vertical axis indicates the amplitude of the heartbeat signal.

The heartbeat interval calculation unit 120 is a processing unit that detects amplitude peaks of the heartbeat signal based on the heartbeat signal data and detects an interval between timings of the detected peaks. The interval between timings at which the amplitude peaks of the heartbeat signal are detected is referred to as a heartbeat interval. Processing of the heartbeat interval calculation unit 120 will be described with reference to FIG. 2. As illustrated in FIG. 2, the heartbeat interval calculation unit 120 detects points R at which the amplitude of the heartbeat signal is equal to or greater than a threshold value, that is, amplitude peaks, and detects an interval between the detected points R, as the heartbeat interval. The heartbeat interval calculation unit 120 outputs data of the detected heartbeat interval to the analysis unit 130. In the following description, the data of the heartbeat interval is referred to as heartbeat interval data.

The analysis unit 130 is a processing unit that performs frequency analysis based on the heartbeat interval data. The analysis unit 130 stores the heartbeat interval data which is obtained from the subject and detected by the heartbeat interval calculation unit 120, for at least a certain period. The analysis unit 130 calculates a power spectral density corresponding to the heartbeat interval, by performing, at predetermined time intervals, frequency analysis of the heartbeat interval data which is obtained during the just previous certain period.

In the following, an example of processing by which the analysis unit 130 calculates the power spectral density corresponding to the heartbeat interval, will be described. The analysis unit 130 generates data of the heartbeat interval that changes with a change over time, based on the heartbeat interval data. In the following description, the data of the heartbeat interval that changes with a change over time is referred to as heartbeat interval change data.

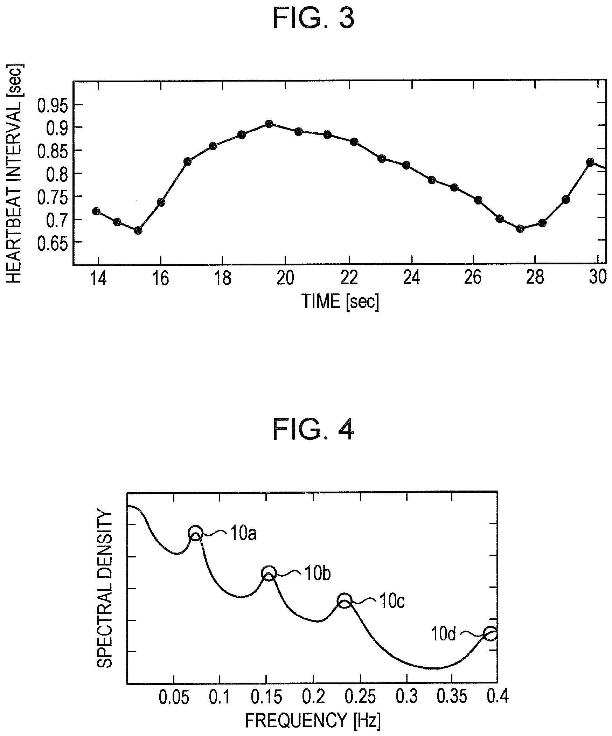

FIG. 3 is a diagram illustrating an example of the heartbeat interval change data. In FIG. 3, the vertical axis indicates the heartbeat interval, and the horizontal axis indicates the time. As illustrated in FIG. 3, the heartbeat interval changes with a change over time.

The analysis unit 130 performs frequency analysis based on the heartbeat interval change data and calculates a relationship between the frequency and the power spectral density. FIG. 4 is a diagram illustrating the relationship between the frequency and the power spectral density. In FIG. 4, the vertical axis indicates the power spectral density, and the horizontal axis indicates the frequency. In the example illustrated in FIG. 4, the power spectral density has maximum values at points 10a, 10b, 10c, and 10d. In the following description, data indicating the relationship between the power spectral density and the frequency is referred to as power spectral density data.

Here, when calculating the relationship between the power spectral density and the frequency, the analysis unit 130 may use any method and may calculate the power spectral density using an autoregressive (AR) model. The AR model is disclosed, for example, in Non-Patent Literature (Shunsuke SATO; Sho KITKAWA; and Tooru KIRYU, Introduction to Biological Signal Processing, Corona Publishing Co., Ltd., January 8, 2004) or the like. The AR model is a model which represents a state at a certain time point by using the linear sum of previous time-series data and has a feature in that a clear maximum point is obtained by using a small amount of data as compared with Fourier transform. The analysis unit 130 may calculate the relationship between the power spectral density and the frequency by Fourier transform.

The p-th order AR model of a time-series $x(s)$ may be represented by Equation (1) using the AR coefficient $a(m)$ that is a weight to a previous value and the error term $e(s)$. In Equation (1), in an ideal state, $e(s)$ corresponds to

white noise.

$$x(s) = \sum_{m=1}^{P} a(m)x(s-m) + e(s) \qquad \cdots(1)$$

It is assumed that p is an identification order, that $f_s$ is a sampling frequency, and that $\varepsilon_p$ is an identification error. Further, it is assumed that the following symbol in Equation 2 is a kth-order AR coefficient.

$$\hat{a}_P(k) \qquad \cdots(2)$$

Then, based on Equations (1) and (2), the power spectral density $P_{AR}(f)$ is represented by Equation (3). The analysis unit 130 calculates the power spectral density data based on Equation (3) and the heartbeat interval change data.

$$P_{AR}(f) = \frac{1}{f_s} \frac{\varepsilon_P}{\left|1 + \sum_{k=1}^{P} \hat{a}_P(k)e^{\frac{-2\pi jkf}{f_k}}\right|^2} \qquad \cdots(3)$$

The evaluation unit 140 is a processing unit that evaluates an arousal level of the subject at each time based on the analysis result of the analysis unit 130. For example, the evaluation unit 140 evaluates the arousal level, based on the maximum value of the power spectral density corresponding to the heartbeat interval obtained by the frequency analysis of the analysis unit 130, and the frequency corresponding to the maximum value of the power spectral density.
In the following, an example of processing by which the evaluation unit 140 evaluates the arousal level will be described. First, the processing by which the evaluation unit 140 evaluates the arousal level based on the maximum value of the power spectral density and the frequency corresponding to the maximum value of the power spectral density, will be described. In the following description, the maximum value of the power spectral density is referred to as a maximum power spectral density. Further, the frequency corresponding to the maximum power spectral density is referred to as a maximum frequency.
The evaluation unit 140 calculates a frequency f that satisfies a relationship of equation (4), as the maximum frequency. The evaluation unit 140 obtains the maximum power spectral density by substituting the maximum frequency into equation (3).

$$\frac{dP_{AR}(f)}{df} = 0 \qquad \cdots(4)$$

The evaluation unit 140 selects a certain maximum power spectral density based on the power spectral density data. In the power spectral density, generally two or more maximum power spectral densities are present. Among the maximum power spectral densities, a maximum power spectral density which is within a frequency range (LF, HF) to be described later and has the maximum peak value, is selected. For example, in FIG. 4, the evaluation unit 140 selects the maximum power spectral density 10a among the maximum power spectral densities 10a to 10d, and observes the selected maximum power spectral density and a change over time in the maximum frequency corresponding to the maximum power spectral density.
For example, the evaluation unit 140 plots, on a graph, the relationship between the observed maximum power spectral density and the maximum frequency corresponding to the maximum power spectral density. Points on the graph that are set by the maximum power spectral density and the maximum frequency are referred to as feature points. The evaluation unit 140 evaluates the arousal level of the subject based on positions of the feature points on the graph.

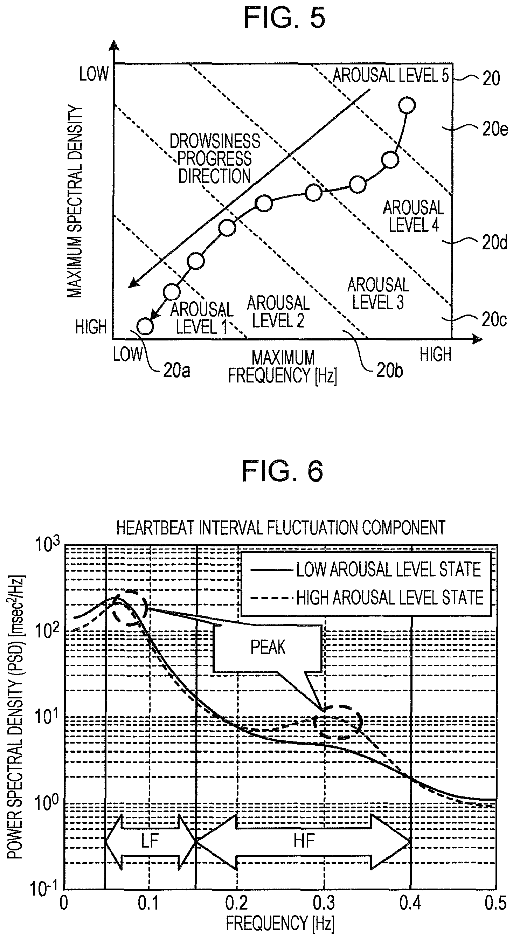

FIG. 5 is a diagram for explaining the processing of evaluating the arousal level. In a graph 20 illustrated FIG. 5, the vertical axis corresponds to the maximum power spectral density. In the graph 20, the maximum power spectral density decreases from the bottom to the top. In the graph 20, the horizontal axis corresponds to the maximum frequency. In the graph 20, the maximum frequency increases from the left to the right. When the maximum frequency decreases and the maximum power spectral density increases, the arousal level of the subject decreases. On the other hand, when the maximum frequency increases and the maximum power spectral density decreases, the arousal level of the subject increases. That is, as the feature points move from the lower left to the upper right, it may be said that the arousal level of the subject moves in a arousal progress direction.

For example, in a case where the positions of the feature points are included in an area 20a, the evaluation unit 140 evaluates the arousal level of the subject as an "arousal level 1". In a case where the positions of the feature points are included in an area 20b, the evaluation unit 140 evaluates the arousal level of the subject as an "arousal level 2". In a case where the positions of the feature points are included in an area 20c, the evaluation unit 140 evaluates the arousal level of the subject as an "arousal level 3". In a case where the positions of the feature points are included in an area 20d, the evaluation unit 140 evaluates the arousal level of the subject as an "arousal level 4". In a case where the positions of the feature points are included in an area 20e, the evaluation unit 140 evaluates the arousal level of the subject as an "arousal level 5".

In the graph illustrated in FIG. 5, as an example, the whole area of the graph 20 is divided into the areas 20a to 20e, and the arousal level of the subject is classified into one of the arousal level 1 to the arousal level 5. Here, the classification level of the arousal level of the subject is not limited thereto. For example, the areas of the graph 20 may be further divided, and the arousal level of the subject may be classified into more detailed levels.

The evaluation unit 140 confirms whether or not the arousal level is less than a predetermined threshold value TH1 by comparing the arousal level with the threshold value TH1. In a case where the arousal level is less than the threshold value TH1, the evaluation unit 140 outputs information indicating a low arousal level state of the subject, to the determination unit 150.

Next, processing by which the evaluation unit 140 evaluates the arousal level of a driver based on feature data of the power spectral density for each frequency range, will be described. The heartbeat interval changes with breathing. That is, the heartbeat interval changes with adjustment of autonomic nerves. Factors of the change include, for example, a change in blood pressure by heartbeat that is called as mayer wave sinus arrhythmia (MWSA), and respiratory sinus arrhythmia (RSA). In a period of the respiratory change in the heartbeat interval data, low frequency (LF) components in the vicinity of 0.05 Hz to 0.15 Hz corresponding to MWSA and high frequency (HF) components in the vicinity of 0.15 Hz to 0.4 Hz corresponding to RSA, are included. Thus, the power spectral density has the following features. FIG. 6 is a diagram for explaining features of frequency bands. In FIG. 6, the horizontal axis indicates frequency and the vertical axis indicates power spectral density. For example, a range of 0.05 Hz to 0.15 Hz corresponds to a frequency range of low frequency (LF), and a range of 0.15 Hz to 0.4 Hz corresponds to a frequency range of high frequency (HF). An active state of a sympathetic nerve is likely to appear in power spectral density components in the frequency range of LF. An active state of a parasympathetic nerve is likely to appear in power spectral density components in the frequency range of HF. In the example of FIG. 6, waveforms of power spectral density distributions are illustrated for frequencies in a high arousal level state and a low arousal level state. As illustrated in FIG. 6, in the high arousal level state, a peak having the maximum power spectral density in the frequency range of HF is higher than that in the low arousal level state. In addition, the high arousal level state has higher power spectral density in the frequency range of HF than that in the low arousal level state. The frequency ranges of LF and HF may be fixed to the above ranges, and may be changed according to age, sex, race, or the like of the subject.

The evaluation unit 140 evaluates the arousal level of the driver based on the feature data of the frequency distribution for each of the frequency ranges of LF and HF. The feature data may be a power spectral density of the maximum point as the peak, or may be a value obtained by integrating the power spectral density in the frequency range. The evaluation unit 140 obtains the arousal level based on the feature data for each of the frequency ranges of LF and HF. For example, the evaluation unit 140 obtains the arousal level based on the fact that the arousal level is higher as the feature data in the frequency range of HF is greater than the feature data in the frequency range of LF. The evaluation unit 140 confirms whether or not the arousal level is equal to or greater than the threshold value TH1 by comparing the arousal level with the threshold value TH1. In a case where the arousal level is not equal to or greater than the threshold value TH1, the evaluation unit 140 outputs information indicating the low arousal level state of the subject, to the determination unit 150. For example, the evaluation unit 140 calculates LF components obtained by integrating the power spectral density in the frequency range of LF and HF components obtained by integrating the power spectral density in the frequency range of HF. In a case where the ratio of the LF components to the HF components is 7:3, the evaluation unit 140 evaluates that the driver is in an arousal state. In a case where the ratio of the LF components to the HF components is 3:7, the evaluation unit 140 outputs information indicating the low arousal level state of the subject, to the determination unit 150.

In the case of receiving the information indicating the low arousal level state of the subject from the evaluation unit 140, the determination unit 150 determines which state the subject is in, within the low arousal level state. That is, the determination unit 150 estimates that which state the subject is in, within the low arousal level state.

Here, the low arousal level state includes a state other than a state where the driver is in a drowsiness state. For example, the low arousal level state includes a drowsiness state and a relaxation state where the driver is relaxed. The drowsiness state is a state where the driver feels drowsiness and attention of the driver is reduced. The relaxation state is a state where the driver does not feel drowsiness and attention of the driver is maintained. In a case where the driver is in the relaxation state, since the driver maintains attention and rapidly responds to a change in situation, variation in the frequency distribution increases. On the other hand, in a case where the driver is in the drowsiness state, since attention of the driver is reduced and response to the change in situation becomes slow, the frequency distribution is biased and the variation in the frequency distribution decreases.

The determination unit 150 determines whether the subject is in a drowsiness state or in a relaxation state, according to a degree of the variation in the frequency distribution that is obtained from the frequency analysis result of the heartbeat interval data by the analysis unit 130.

In the following, an example of the processing by which the determination unit 150 estimates whether the subject is in a drowsiness state or in a relaxation state according to the degree of the variation in the frequency distribution, will be described. First, the processing by which the determination unit 150 estimates whether the subject is in a drowsiness state or in a relaxation state by using entropy as the degree of the variation in the frequency distribution, will be described.

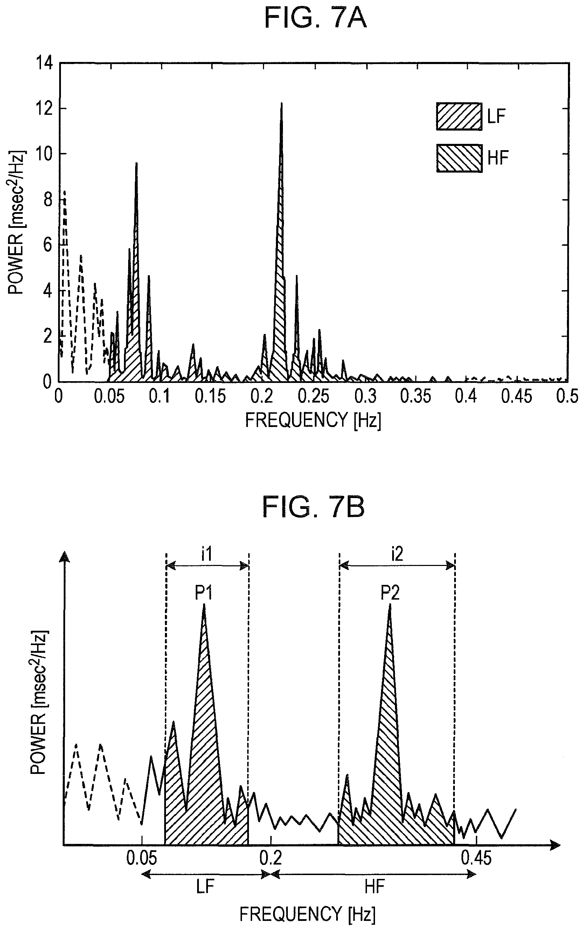

FIG. 7A is a diagram illustrating an example of the frequency distribution. In the example of FIG. 7A, the frequency distribution in the frequency range of LF (0.05 Hz to 0.15 Hz) and the frequency distribution in the frequency range of HF (0.15 Hz to 0.4 Hz) are represented as hatched portions. The determination unit 150 divides the frequency distribution into portions with a predetermined frequency bandwidth, and obtains an integral value of the power spectral density for each frequency bandwidth by integrating the power spectral density in the frequency bandwidth. The frequency bandwidth may be any one as long as the integral value approximately indicating the frequency distribution in the target frequency range may be obtained, and is, for example, 0.01 Hz. The determination unit 150 obtains a ratio of the integral value of the power spectral density in each frequency bandwidth to the integral value of the power spectral density in the whole frequency bandwidth. The ratio of the integral value of the power spectral density for each frequency bandwidth is an appearance probability in each frequency bandwidth. The appearance probability may be obtained as a ratio of the integral value of the power spectral density in the frequency bandwidth to the integral value of the power spectral density in the frequency ranges of LF and HF.

The appearance probability $Pi$ in the frequency bandwidth $i$ may be represented by equation (5-1) using power spectral density (PSD) function representing the power spectral density with respect to the frequency $f$. Entropy $H$ is represented by equation (5-2). Here, $Pi$ is a probability distribution, $i=1, 2,..., n$, and $\Delta f=0.05$ Hz. $\Delta f$ may be a value slightly less than or greater than 0.05 Hz. Here, preferably, the lower limit of the integral is 0.05 Hz which is the lower limit of LF of heartbeat fluctuation frequencies, and the upper limit of the integral is 0.4 Hz which is the upper limit of HF of the heartbeat fluctuation frequencies. The determination unit 150 calculates the entropy $H$ from the appearance probability $Pi$ in the frequency bandwidth $i$ using equations (5-1) and (5-2).

$$Pi = \int_{\Delta f x i}^{\Delta f x i + \Delta f} PSD(f) df \quad \cdots(5\text{-}1)$$

$$H = -Pi \sum \log_2 Pi \quad \cdots(5\text{-}2)$$

In a case where the calculated entropy $H$ is less than a predetermined threshold value TH2, the determination unit 150 estimates that the subject is in a relaxation state, and in a case where the entropy $H$ is equal to or greater than the threshold value TH2, the determination unit 150 estimates that the subject is in a drowsiness state. The threshold value TH2 may be a fixed value or may be changed dynamically. For example, a standard threshold value TH2 may be stored in advance for each sex and age group, and the threshold value TH2 may be set according to the sex or the age group of the subject. In the present example, the initial value of the threshold value TH2 is determined, and the threshold value TH2 is dynamically changed by the setting unit 160 to be described later. The initial value may be one value or may be set according to the sex or the age group of the subject. In a case where it is estimated that the subject is in a drowsiness state, the determination unit 150 outputs information indicating that the subject is estimated to be in a drowsiness state, to the warning unit 170.

Further, the determination unit 150 may calculate the entropy in a predetermined frequency bandwidth with the peak as a reference within a predetermined frequency range of the frequency distribution. For example, the determination unit 150 may calculate the entropy $H$ in a predetermined frequency bandwidth with the peak as a center for each of the frequency ranges of LF and HF, from the frequency distribution. FIG. 7B is a diagram illustrating an example of a frequency range in the frequency distribution that is used for calculating the entropy. In the example of FIG. 7B, the peak P1 of the power spectral density is illustrated in the frequency range of LF, and the peak P2 of the power spectral density is illustrated in the frequency range of HF. For example, the determination unit 150 calculates the appearance probability for a range i1 of the predetermined frequency bandwidth with the peak P1 as a center by using equation (5-1), and calculates the entropy for the range i1 by using (5-2). In addition, the determination unit 150 calculates the appearance probability for a range i2 of the predetermined frequency bandwidth with the peak P2 as a center by using equation (5-1), and calculates the entropy for the range i2 by using (5-2). In the example of FIG. 7B, the range i1 and the range i2 in the frequency distribution are represented as hatched portions. The predetermined frequency bandwidth may be any one as long as the degree of the variation in the frequency distribution may be specified in the predetermined frequency bandwidth. For example, the predetermined frequency bandwidth may be set to approximately a half of the frequency range. In addition, the predetermined frequency bandwidth may be individually determined for each of the frequency ranges of LF and HF. For example, the predetermined frequency bandwidth with the peak as a center in the frequency range of LF is set to 0.05 Hz which is a half of the frequency range of LF. The predetermined frequency bandwidth with the peak as a center in the frequency range of HF is set to 0.125 Hz which is a half of the frequency range of HF.

In a case where the entropy in the predetermined frequency bandwidth with the peak as a reference is less than the threshold value TH2, the determination unit 150 estimates that the subject is in a relaxation state, and in a case where the entropy is equal to or greater than the threshold value TH2, the determination unit 150 estimates that the subject is in a drowsiness state. For example, in a case where the entropy in the predetermined frequency bandwidth within each of the frequency ranges of LF and HF is less than a predetermined threshold value, the determination unit 150 estimates that the subject is in a relaxation state. In a case where the entropy in the predetermined frequency bandwidth within one or both of the frequency ranges of LF and HF is not less than the predetermined threshold value, the determination unit 150 estimates that the subject is in a drowsiness state. The difference in the frequency distribution between the relaxation state and the drowsiness state is likely to appear in the power spectral density in the vicinity of the peak. A portion of the power spectral density that is away from the peak is likely to be a noise component when estimating the state of the subject. Thus, the determination unit 150 estimates the state of the subject based on the entropy in the predetermined frequency bandwidth with the peak as a reference within the predetermined frequency range of the frequency distribution. Thereby, the determination unit 150 may estimate whether the subject is in a relaxation state or in a drowsiness state with high accuracy. In the case of driving in the real world, when the subject is in a drowsiness state, an operation for trying to stay awake in order to shake off drowsiness and consciousness for arousal (so-called an arousal effort, for example, an operation such as shaking a head, sitting again, or shaking a body and enhancement of consciousness for trying to stay awake), appear. Due to the influence, a variation in the respiratory component change is slightly observed in the vicinity of RSA, and a variation in the blood pressure change is slightly observed in the vicinity of MWSA. On the contrary, at the time of relaxation, the respiratory component and the blood pressure change in a stable convergence direction, without occurrence of such a state. Thus, at the time of drowsiness, the entropy in the vicinity of RSA and MWSA becomes higher than that at the time of relaxation. Based on the tendency, it is possible to distinguish whether the subject is in a relaxation state or in a drowsiness state.

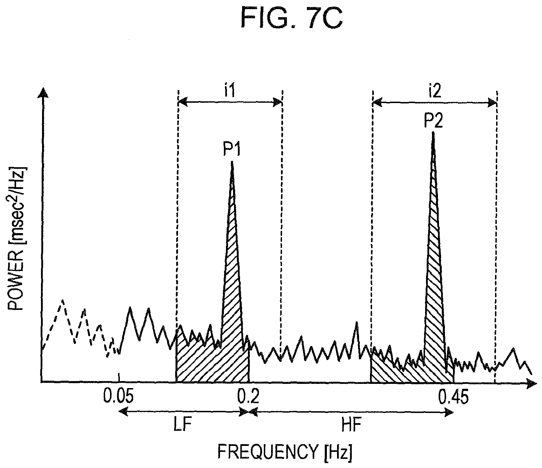

On the other hand, in some cases, the range of the predetermined frequency bandwidth with the peak as a reference may exceed a predetermined frequency range. For example, in a case where the peaks in the frequency ranges of LF and HF are respectively present at the end portions of the frequency ranges of LF and HF, in some cases, the ranges i1 and i2 of the predetermined frequency bandwidth with the peak as a center may exceed the frequency ranges of LF and HF. FIG. 7C is a diagram illustrating an example of a frequency range in the frequency distribution that is used for calculating the entropy. In the example of FIG. 7C, the range i1 exceeds the frequency range of LF. The range i2 exceeds the frequency range of HF. In a case where the range of the predetermined frequency bandwidth with the peak as a reference exceeds the predetermined frequency range, the determination unit 150 may calculate the entropy in the range of the predetermined frequency bandwidth with the peak as a reference and in the frequency range of the frequency distribution. For example, in the example of FIG. 7C, the determination unit 150 calculates the entropy for the range in the frequency range of LF within the range i1. In addition, the determination unit 150 calculates the entropy for the range in the frequency range of HF within the range i2. In the example of FIG. 7C, portions in the frequency ranges of LF and HF within the range i1 and the range i2 of the frequency distribution are patterned. The difference between the relaxation state of the subject and the drowsiness state of the subject is likely to appear in the frequency ranges of LF and HF. Thus, even in a case where the range of the predetermined frequency bandwidth with the peak as a reference exceeds the frequency ranges of LF and HF, the determination unit 150 calculates the entropy in the range of the predetermined frequency bandwidth with the peak as a reference and in the frequency ranges of LF and HF of the frequency distribution. Thus, the determination unit 150 may accurately estimate whether the subject is in a relaxation state or in a drowsiness state.

Next, the processing by which the determination unit 150 estimates whether the subject is in a drowsiness state or in a relaxation state by using variance as the degree of the variation in the frequency distribution, will be described.

The determination unit 150 obtains a variance of the power spectral density in a certain section of the frequency distribution. The certain section may be the entire frequency range or a specific frequency range such as the frequency ranges of LF and HF (0.05 Hz to 0.4 Hz). In a case where n frequencies $f_1, f_2,..., f_n$ in the certain section are given, the determination unit 150 represents an average value of the n frequencies by expression (6). In addition, the variance $S^2$ is represented by equation (7).

$$\overline{f} = f_1 + f_2 + \cdots + f_n \qquad \cdots (6)$$

$$s^2 = \frac{1}{n-1}\left(\left(f_1 - \overline{f}\right)^2 + \left(f_2 - \overline{f}\right)^2 + \cdots + \left(f_n - \overline{f}\right)^2\right) \quad \cdots (7)$$

In a case where the calculated variance $S^2$ is less than a predetermined threshold value TH2, the determination unit 150 estimates that the subject is in a relaxation state, and in a case where the variance $S^2$ is equal to or greater than the threshold value TH2, the determination unit 150 estimates that the subject is in a drowsiness state. The threshold value TH2 may be a fixed value or may be changed dynamically. In the present example, the initial value of the threshold value TH2 is determined, and the threshold value TH2 is dynamically changed by the setting unit 160 to be described later. In a case where it is estimated that the subject is in a drowsiness state, the determination unit 150 outputs information indicating that the subject is estimated to be in a drowsiness state, to the warning unit 170.

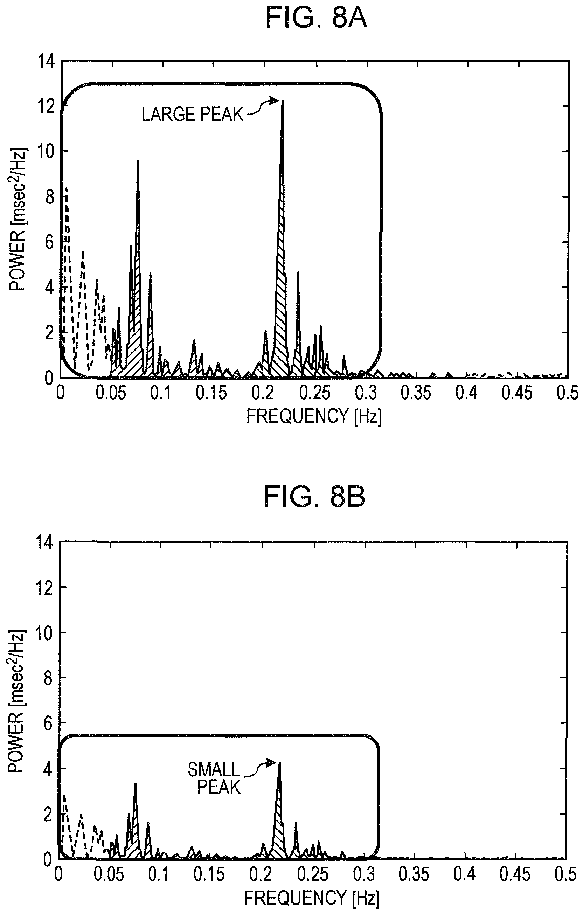

Here, the power spectral density of the frequency distribution also changes depending on condition or the like of the subject. For example, in a case where the subject is in a bad condition state, the overall power spectral density of the frequency distribution decreases as compared with the case where the subject is in a normal state (healthy state). FIG. 8A is a diagram illustrating an example of the frequency distribution in a case where the subject is in a normal condition state. FIG. 8B is a diagram illustrating an example of the frequency distribution in a case where the subject is in a bad condition state. The overall power spectral density of the frequency distribution illustrated in FIG. 8B is lower than that of the frequency distribution illustrated in FIG. 8A. In a case where the subject is in a bad condition state, the frequency distribution is unlikely to be biased. In addition, when the subject is in a bad condition state, attention is likely to decrease.

Thus, in a case where the overall power spectral density of the frequency distribution is low, the determination unit 150 may estimate that the subject is in a drowsiness state, regardless of the degree of variation in the frequency distribution. For example, in a case where the integral value or the peak of the power spectral density is equal to or less than a predetermined threshold value TH3, the determination unit 150 estimates that the subject is in a drowsiness state, regardless of the degree of variation in the frequency distribution. For example, the threshold value TH3 is set to a value that may be regarded as a bad condition state, by obtaining the condition state and the power spectral density of the frequency distribution for a large number of subjects.

The setting unit 160 sets the threshold value TH2 which is used for determination between the relaxation state and the drowsiness state. Typically, when the subject drives a vehicle, a drowsiness level is low. On the other hand, as a driving time increases, the driver is likely to feel drowsiness. Thus, the setting unit 160 sets the threshold value TH2 based on the frequency distribution after the elapse of predetermined period of time from the time when the subject drives the vehicle. The predetermined period of time may be any period of time as long as the frequency distribution when the subject is in a stable state after driving the vehicle may be acquired. The predetermined period of time may be, for example, five minutes. For example, in a case where the entropy is used as the degree of variation in the frequency distribution, the setting unit 160 calculates the entropy of the frequency distribution after the elapse of predetermined period of time from the time when the subject drives the vehicle. The setting unit 160 sets the threshold value TH2 to a value less than the calculated entropy. For example, the setting unit 160 sets the threshold value TH2 to a value corresponding to 70% of the calculated entropy. Similarly, for example, in a case where the variance is used as the degree of variation in the frequency distribution, the setting unit 160 calculates the variance $S^2$ of the frequency distribution after the elapse of predetermined period of time from the time when the subject drives the vehicle. The setting unit 160 sets the threshold value to a value less than the calculated variance $S^2$. For example, the setting unit 160 sets the threshold value TH2 to a value corresponding to 70% of the calculated variance $S^2$. Accordingly, the setting unit 160 may set the threshold value TH2 according to the driver, and thus the state of the driver may be identified with high accuracy.

The warning unit 170 is a processing unit that receives the estimation result from the determination unit 150 and outputs a warning according to the estimation result. Specifically, when receiving information indicating that the subject is estimated to be in a drowsiness state, the warning unit 170 outputs a warning to the subject. The warning unit 170 may output a warning using sound or a warning using video by a display provided in the vehicle.

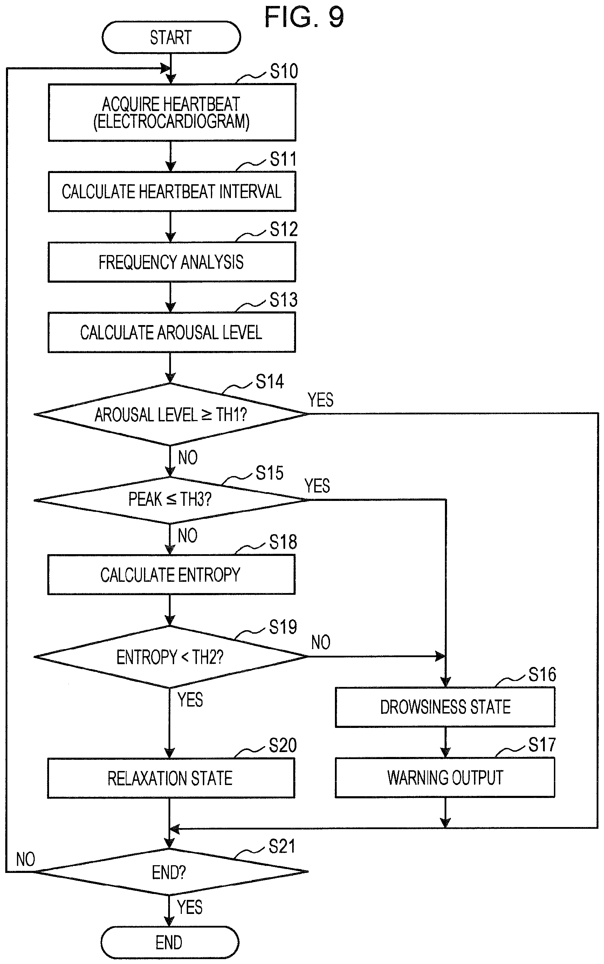

Next, a flow of execution of drowsiness detection processing for detecting drowsiness by the drowsiness detection apparatus 100 according to the present example, will be described. FIG. 9 is a flowchart illustrating an example of a procedure of the drowsiness detection processing. The drowsiness detection processing is executed at a predetermined timing, for example, at a timing when an engine of the vehicle is started and an instruction to start the processing is received from a control unit of the vehicle.

As illustrated in FIG. 9, the sensor 110 of the drowsiness detection apparatus 100 detects the heartbeat signal of the subject and acquires the heartbeat signal data (S10). The heartbeat interval calculation unit 120 detects amplitude peaks of the heartbeat signal based on the heartbeat signal data and calculates an interval between timings of the detected peaks (S11).

The analysis unit 130 calculates a power spectral density corresponding to the heartbeat interval, by performing frequency analysis of the heartbeat interval data which is obtained during the just previous certain period (S12). The evaluation unit 140 derives the arousal level, based on the maximum value of the power spectral density corresponding to the heartbeat interval obtained by the frequency analysis, and the frequency corresponding to the maximum value of the power spectral density (S13).

The evaluation unit 140 evaluates whether or not the derived arousal level is equal to or greater than a threshold value TH1 (S14). In a case where the arousal level is equal to or greater than the threshold value TH1 (YES in S14), the process proceeds to S21 to be described later.

On the other hand, in a case where the arousal level is not equal to or greater than the threshold value TH1 (NO in S14), the determination unit 150 determines whether the overall power spectral density of the frequency distribution is in a low state. For example, the determination unit 150 determines whether the peak of the power spectral density is equal to or less than the threshold value TH3 (S15). In a case where the peak of the power spectral density is equal to or less than the threshold value TH3 (YES in S15), since the overall power spectral density of the frequency distribution is in a low state, the determination unit 150 estimates that the subject is in a drowsiness state (S16). The warning unit 170 outputs a warning to the subject (S17), and the process proceeds to S21 to be described later.

On the other hand, in a case where the peak of the power spectral density is not equal to or less than the threshold value TH3 (NO in S15), the determination unit 150 calculates the entropy H of the power spectral density (S18). For example, the determination unit 150 calculates the entropy in a predetermined frequency bandwidth with the peak as a reference within a predetermined frequency range of the frequency distribution. For example, the determination unit 150 specifies the peak of the power spectral density for each of the frequency ranges of LF and HF from the frequency distribution. The determination unit 150 calculates the entropy H in the predetermined frequency bandwidth with the peak as a center for each of the frequency ranges of LF and HF.

The determination unit 150 determines whether or not the entropy H is less than the threshold value TH2 (S19). In a case where the entropy H is not less than the threshold value TH2 (NO in S19), the process proceeds to S16. For example, in a case where the entropy in the predetermined frequency bandwidth within each of the frequency ranges of LF and HF is less than a predetermined threshold value, the determination unit 150 causes the process to proceed to S20. In a case where the entropy in the predetermined frequency bandwidth within one or both of the frequency ranges of LF and HF is not less than the predetermined threshold value, the determination unit 150 causes the process to proceed to S16. Accordingly, the subject is estimated to be in a drowsiness state and a warning is output to the subject.

On the other hand, in a case where the entropy H is less than the threshold value TH2 (YES in S19), the determination unit 150 estimates that the subject is in a relaxation state (S20), and the process proceeds to S21 to be described later.

The analysis unit 130 confirms whether or not processing end is instructed (S21). For example, in a case where the engine of the vehicle stops and an instruction for processing end is received from the control unit of the vehicle, the analysis unit 130 confirms that the processing end is instructed. In a case where the processing end is instructed (YES in S21), the processing is ended.

On the other hand, in a case where the processing end is not instructed (NO in S21), the process proceeds to S10 described above.

As described above, the drowsiness detection apparatus 100 according to the present example performs frequency analysis on the heartbeat data obtained from the subject. The drowsiness detection apparatus 100 estimates whether the subject is in a drowsiness state or in a relaxation state according to the degree of variation in the frequency distribution obtained from the frequency analysis result. Accordingly, the drowsiness detection apparatus 100 may estimate the drowsiness state of the subject with high accuracy.

In addition, the drowsiness detection apparatus 100 according to the present example calculates the entropy or the variance from the frequency distribution. In a case where the calculated entropy or the calculated variance is less than a predetermined threshold value (threshold value TH2), the drowsiness detection apparatus 100 estimates that the subject is in a relaxation state. In a case where the calculated entropy or the calculated variance is equal to or greater than the threshold value (threshold value TH2), the drowsiness detection apparatus 100 estimates that the subject is in a drowsiness state. Accordingly, the drowsiness detection apparatus 100 may determine whether the variation in the frequency distribution is large or small, and thus it is possible to estimate the drowsiness state of the subject with high accuracy.

Further, the drowsiness detection apparatus 100 according to the present example calculates the entropy or the variance from the frequency distribution after the elapse of predetermined period of time from the time when the subject drives the vehicle, and sets the threshold value (threshold value TH2) to a value greater than the calculated entropy or the calculated variance. Accordingly, the drowsiness detection apparatus 100 may set the threshold value TH2 according to the driver, and thus the state of the driver may be identified with high accuracy.

Further, the drowsiness detection apparatus 100 according to the present example determines the arousal level of the subject from the power spectral density of the heartbeat interval calculated from the heartbeat data. In a case where the arousal level of the subject is equal to or less than a predetermined value (threshold value TH1), the drowsiness detection apparatus 100 estimates whether the subject is in a drowsiness state or in a relaxation state according to the degree of variation in the frequency distribution. Accordingly, the drowsiness detection apparatus 100 may estimate whether the subject having a low arousal level is in a drowsiness state or in a relaxation state.

In a case where the integral value or the peak of the power spectral density is equal to or less than a predetermined value, the drowsiness detection apparatus 100 according to the present example estimates that the subject is in a drowsiness state, regardless of the degree of variation in the frequency distribution. Accordingly, the drowsiness detection apparatus 100 outputs a warning when the subject is in a bad condition state and the arousal level of the subject is low, and thus occurrence of an accident may be reduced.

In addition, the drowsiness detection apparatus 100 according to the present example performs frequency analysis on the heartbeat data obtained from the subject. The drowsiness detection apparatus 100 calculates the entropy in the predetermined frequency bandwidth with the peak as a reference within the predetermined frequency range of the frequency distribution obtained from the frequency analysis result. In a case where the calculated entropy is less than a predetermined threshold value, the drowsiness detection apparatus 100 estimates that the subject is in a relaxation state, and in a case where the entropy is equal to or greater than the threshold value, the drowsiness detection apparatus 100 estimates that the subject is in a drowsiness state. Accordingly, the drowsiness detection apparatus 100 may estimate whether the subject is in a relaxation state or in a drowsiness state.

Further, the drowsiness detection apparatus 100 according to the present example calculates the entropy in the predetermined frequency bandwidth with the peak as a center for each of the frequency ranges of LF and HF, from the frequency distribution. The drowsiness detection apparatus 100 estimates whether the subject is in a drowsiness state or in a relaxation state based on the calculated entropy in the predetermined frequency bandwidth within each of the frequency ranges of LF and HF. The difference between the relaxation state of the subject and the drowsiness state of the subject is likely to appear in the frequency ranges of LF and HF. Therefore, the drowsiness detection apparatus 100 may estimate whether the subject is in a relaxation state or in a drowsiness state with high accuracy.

In a case where the entropy in the predetermined frequency bandwidth within each of the frequency ranges of LF and HF is less than a predetermined threshold value, the drowsiness detection apparatus 100 according to the present example estimates that the subject is in a relaxation state. In a case where the entropy in the predetermined frequency bandwidth within one or both of the frequency ranges of LF and HF is not less than the predetermined threshold value, the drowsiness detection apparatus 100 estimates that the subject is in a drowsiness state. Accordingly, the drowsiness detection apparatus 100 may estimate whether the subject is in a relaxation state or in a drowsiness state with high accuracy.

Further, the drowsiness detection apparatus 100 according to the present example calculates the entropy in the range of the predetermined frequency bandwidth with the peak as a reference and in the frequency range of the frequency distribution. Accordingly, the drowsiness detection apparatus 100 may estimate whether the subject is in a relaxation state or in a drowsiness state with higher accuracy.

Second Example

In the above description, the example related to the apparatus disclosed herein is described. The technology disclosed herein may be applied in various different forms in addition to the above-described example. Hereinafter, another example included in the embodiments disclosed herein will be described.

For example, in the above example, the case of estimating whether the subject is in a drowsiness state or in a relaxation state based on the degree of overall variation in the frequency distribution, is described. However, the apparatus disclosed herein is not limited thereto. The determination unit 150 may estimate whether the subject is in a drowsiness state or in a relaxation state based on the degree of variation in a specific frequency range of the frequency distribution. For example, the determination unit 150 calculates the entropy or the variance for each of the frequency ranges of LF and HF, from the frequency distribution. The determination unit 150 may estimate whether the subject is in a drowsiness state or in a relaxation state based on the calculated entropy or the calculated variance in each of the frequency ranges of LF and HF. For example, in a case where the entropy or the variance in each of the frequency ranges of LF and HF is equal to or greater than a predetermined threshold value, the determination unit 150 may estimate that the subject is in a relaxation state. In a case where the entropy or the variance in one or both of the frequency ranges of LF and HF is not equal to or greater than the predetermined threshold value, the determination unit 150 may estimate that the subject is in a drowsiness state. The determination unit 150 may estimate whether the subject is in a drowsiness state or in a relaxation state based on the degree of variation in the frequency range of LF or HF.

For example, in the above example, the case of estimating that the subject is in a drowsiness state when the peak of the power spectral density is equal to or less than the threshold value TH3 without calculating the entropy or the variance, is described. However, the apparatus disclosed herein is not limited thereto. Even in a case where the peak of the power spectral density is equal to or less than the threshold value TH3, the determination unit 150 may calculate the entropy or the variance, and estimate whether the subject is in a drowsiness state or in a relaxation state based on the entropy or the variance.

In addition, the components of the apparatus illustrated in FIG. 1 are functionally conceptual and may be not physically configured as illustrated in FIG. 1. That is, specific forms of separation and combination of each unit are not limited to that illustrated in FIG. 1, and a configuration in which all or some of the units are functionally or physically separated or combined in an arbitrary unit according to various types of loads or usage environments, may be made. For example, each processing unit of the heartbeat interval calculation unit 120, the analysis unit 130, the evaluation unit 140, the determination unit 150, and the setting unit 160 may be combined as appropriate. Further, each processing unit may be divided into a plurality of processing units by functions. Furthermore, all or any of various processing functions performed by each processing unit may be implemented by a CPU or a program that is analyzed and executed by the CPU, or may be implemented as hardware by a wired logic.

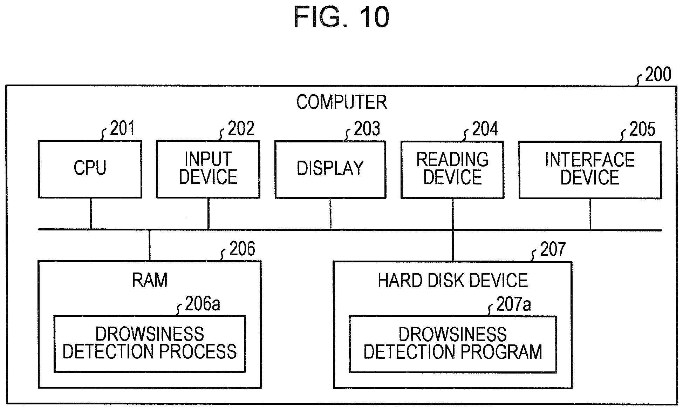

Next, an example of a computer that executes a drowsiness detection program for realizing the same function as that of the drowsiness detection apparatus 100 described in the example, will be described. FIG. 10 is a diagram illustrating an example of a computer that executes the drowsiness detection program.

As illustrated in FIG. 10, the computer 200 includes a CPU 201 that executes various calculation processing, an input device 202 that receives input of data from a user, and a display 203. The computer 200 also includes a reading device 204 that reads a program or the like from a storage medium and an interface device 205 that exchanges data with another computer via a network. In addition, the computer 200 also includes a RAM 206 that temporarily stores various kinds of information, and a hard disk device 207. Each of the devices 201 to 207 is connected to a bus 208.

The hard disk device 207 stores, for example, a drowsiness detection program 207a. The CPU 201 reads the drowsiness detection program 207a and loads the program into the RAM 206. The drowsiness detection program 207a functions as a drowsiness detection process 206a. The drowsiness detection process 206a corresponds to, for example, the heartbeat interval calculation unit 120, the analysis unit 130, the evaluation unit 140, the determination unit 150, and the setting unit 160.

The drowsiness detection program 207a may not be stored in the hard disk device 207 from the beginning. For example, each program may be stored in a "portable physical medium" such as a flexible disk (FD), a CD-ROM, a DVD disk, a magneto-optical disk, or an IC card, which is inserted into the computer 200. The computer 200 may read and execute the drowsiness detection program 207a from the medium.

**Claims**

1. A computer-readable medium storing a drowsiness detection program that causes a computer to execute a process, the process comprising:

   performing frequency analysis on heartbeat data obtained from a subject; and
   determining a state of the subject by calculating entropy in a predetermined frequency bandwidth with a peak as a reference, where the peak is within a predetermined frequency range of frequency distribution obtained from the frequency analysis result, determining that the subject is in a relaxation state in a case where the calculated entropy is less than a predetermined threshold value, and determining that the subject is in a drowsiness state in a case where the entropy is equal to or greater than the threshold value.

2. The medium according to claim 1,
   wherein the determining of the state of the subject includes calculating the entropy in the predetermined frequency bandwidth with the peak as a center for each frequency range of predetermined low frequency (LF) and predetermined high frequency (HF) from the frequency distribution, and determining whether the subject is in a drowsiness state or in a relaxation state based on the calculated entropy in the predetermined frequency bandwidth of LF and HF.

3. The medium according to claim 2,
   wherein the determining of the state of the subject includes estimating that the subject is in a relaxation state in a case where the entropy in the predetermined frequency bandwidth within each of the frequency ranges of LF and HF is less than a predetermined threshold value, and determining that the subject is in a drowsiness state in a case where the entropy in the predetermined frequency bandwidth within one or both of the frequency ranges of LF and HF is equal to or greater than the predetermined threshold value.

4.  The medium according to claim 1,
    wherein the determining of the state of the subject includes calculating the entropy in the range of the predetermined frequency bandwidth with the peak as a reference, where the bandwidth is within the frequency range of the frequency distribution.

5.  A drowsiness detection method that causes a computer to execute a process, the process comprising:

    performing frequency analysis on heartbeat data obtained from a subject; and
    determining a state of the subject by calculating entropy in a predetermined frequency bandwidth with a peak as a reference, where the peak is within a predetermined frequency range of frequency distribution obtained from the frequency analysis result, determining that the subject is in a relaxation state in a case where the calculated entropy is less than a predetermined threshold value, and determining that the subject is in a drowsiness state in a case where the entropy is equal to or greater than the threshold value.

6.  A drowsiness detection apparatus comprising:

    an analysis unit that performs frequency analysis on heartbeat data sequentially obtained from a subject; and
    a determining unit that determines a state of the subject by calculating entropy in a predetermined frequency bandwidth with a peak as a reference, where the peak is within a predetermined frequency range of frequency distribution obtained from the frequency analysis result, wherein determining that the subject is in a relaxation state if the calculated entropy is less than a predetermined threshold value, and determining that the subject is in a drowsiness state if the entropy is equal to or greater than the threshold value.

# FIG. 1

# FIG. 2

# FIG. 3

# FIG. 4

## FIG. 5

## FIG. 6

# FIG. 7A

# FIG. 7B

# FIG. 7C

## FIG. 8A

## FIG. 8B

## FIG. 9

```
                    ┌─────────────┐
                    │    START    │
                    └─────────────┘
                           │
                           ▼              ～ S10
              ┌──────────────────────────┐
              │    ACQUIRE HEARTBEAT      │
              │   (ELECTROCARDIOGRAM)     │
              └──────────────────────────┘
                           │              ～ S11
              ┌──────────────────────────┐
              │ CALCULATE HEARTBEAT INTERVAL │
              └──────────────────────────┘
                           │              ～ S12
              ┌──────────────────────────┐
              │    FREQUENCY ANALYSIS     │
              └──────────────────────────┘
                           │              ～ S13
              ┌──────────────────────────┐
              │   CALCULATE AROUSAL LEVEL │
              └──────────────────────────┘
                           │              ～ S14
                    ◇───────────────◇         YES
                    AROUSAL LEVEL ≥ TH1?─────────┐
                    ◇───────────────◇           │
                           │ NO                  │
                           ▼              ～ S15 │
                    ◇───────────────◇     YES    │
                       PEAK ≤ TH3?────────┐      │
                    ◇───────────────◇     │      │
                           │ NO           │      │
                           ▼       ～ S18  │      │
              ┌──────────────────────────┐│      │
              │     CALCULATE ENTROPY     ││      │
              └──────────────────────────┘│      │
                           │       ～ S19  │      │
                    ◇───────────────◇  NO  │      │
                     ENTROPY < TH2?─────────►     │
                    ◇───────────────◇     │      │
                           │ YES          ▼      │
                                   ～ S16 ┌──────────────┐
                                         │ DROWSINESS STATE │
                                         └──────────────┘
                           │       ～ S20 │      ～ S17
              ┌──────────────────────┐  ┌──────────────┐
              │   RELAXATION STATE    │  │ WARNING OUTPUT │
              └──────────────────────┘  └──────────────┘
                           │              │
                           ▼              │
                    ◇───────────────◇ ～ S21
          NO        │     END?      │◄─────────────┘
          ┌─────────◇───────────────◇
          │                │ YES
          │                ▼
          │         ┌─────────────┐
          │         │     END     │
          │         └─────────────┘
```

# FIG. 10

COMPUTER _200

| CPU _201 | INPUT DEVICE _202 | DISPLAY _203 | READING DEVICE _204 | INTERFACE DEVICE _205 |

RAM _206
DROWSINESS DETECTION PROCESS _206a

HARD DISK DEVICE _207
DROWSINESS DETECTION PROGRAM _207a

Europäisches
Patentamt

European
Patent Office

Office européen
des brevets

# EUROPEAN SEARCH REPORT

Application Number

EP 17 18 1158

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/275847 A1 (KARASUDANI AYU [JP]) 5 November 2009 (2009-11-05) * paragraphs [0027] - [0059]; figures 1-6, * | 1-6 | INV. A61B5/18 ADD. A61B5/024 A61B5/04 A61B5/0456 |
| Y | US 2015/164358 A1 (MOORMAN J RANDALL [US] ET AL) 18 June 2015 (2015-06-18) * paragraphs [0039] - [0041], [0087] - [0094], [0109] * | 1-6 | |
| A | Milan Palus ET AL: "ENTROPIES, PARTITIONINGS AND HEART RATE VARIABILITY", Activitas Nervosa Superior, 1 June 2009 (2009-06-01), pages 65-72, XP055231642, Prague DOI: 10.1007/BF03380037 Retrieved from the Internet: URL:https://rd.springer.com/article/10.1007%2FBF03380037 * the whole document * | 1,5,6 | |
| A | US 2016/113538 A1 (CHIU HUNG-CHIH [TW] ET AL) 28 April 2016 (2016-04-28) * paragraphs [0014], [0018], [0087]; claim 1; figures 1-2 * | 1,5,6 | TECHNICAL FIELDS SEARCHED (IPC) A61B |
| A | US 2014/378857 A1 (MASUDA YUTA [JP] ET AL) 25 December 2014 (2014-12-25) * paragraphs [0002], [0036] - [0057]; figures 1-8,10 * | 1,5,6 | |
| A | US 2013/043886 A1 (MASE ATSUSHI [JP] ET AL) 21 February 2013 (2013-02-21) * paragraphs [0004] - [0007], [0013] - [0027], [0073], [0053], [0099] - [0133]; figures 12,13,14,16 * | 1,5,6 | |

The present search report has been drawn up for all claims

| Place of search | Date of completion of the search | Examiner |
|---|---|---|
| Berlin | 20 December 2017 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 18 1158

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2017

| Patent document cited in search report | | | Publication date | Patent family member(s) | | | Publication date |
|---|---|---|---|---|---|---|---|
| US 2009275847 | A1 | | 05-11-2009 | EP | 2087841 | A1 | 12-08-2009 |
| | | | | JP | 5383200 | B2 | 08-01-2014 |
| | | | | JP | WO2008065724 | A1 | 04-03-2010 |
| | | | | US | 2009275847 | A1 | 05-11-2009 |
| | | | | WO | 2008065724 | A1 | 05-06-2008 |
| US 2015164358 | A1 | | 18-06-2015 | US | 2010056940 | A1 | 04-03-2010 |
| | | | | US | 2015164358 | A1 | 18-06-2015 |
| | | | | WO | 2008128034 | A1 | 23-10-2008 |
| US 2016113538 | A1 | | 28-04-2016 | TW | 201616436 | A | 01-05-2016 |
| | | | | US | 2016113538 | A1 | 28-04-2016 |
| US 2014378857 | A1 | | 25-12-2014 | EP | 2829230 | A1 | 28-01-2015 |
| | | | | JP | 5831622 | B2 | 09-12-2015 |
| | | | | JP | WO2013140525 | A1 | 03-08-2015 |
| | | | | US | 2014378857 | A1 | 25-12-2014 |
| | | | | WO | 2013140525 | A1 | 26-09-2013 |
| US 2013043886 | A1 | | 21-02-2013 | EP | 2537462 | A1 | 26-12-2012 |
| | | | | JP | 5709017 | B2 | 30-04-2015 |
| | | | | JP | WO2011099600 | A1 | 17-06-2013 |
| | | | | US | 2013043886 | A1 | 21-02-2013 |
| | | | | WO | 2011099600 | A1 | 18-08-2011 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007289540 A

- JP 7108847 A

**Non-patent literature cited in the description**

- **SHUNSUKE SATO ; SHO KITKAWA ; TOORU KIRYU.** Introduction to Biological Signal Processing. Corona Publishing Co., Ltd, 08 January 2004